# FASCICULE DE BREVET EUROPEEN

(11) **EP 0 646 193 B1**
(45) Date de publication et mention de la délivrance du brevet: **24.06.1998**
(21) Numéro de dépôt: 94913155.1
(22) Date de dépôt: 11.04.1994
(51) Int. Cl.: D04H 13/00

(54) **MATERIAU PLAN COMPOSITE COMPORTANT UN FILM PERFORE ET DES FIBRES THERMOPLASTIQUES, UTILISATION ET PROCEDE DE FABRICATION**
VERBUND-MATERIAL MIT PERFORIERTER FOLIE UND THERMOPLASTISCHEN FASERN, DESSEN VERWENDUNG UND VERFAHREN ZUR HERSTELLUNG
PLANE COMPOSITE MATERIAL COMPRISING A PIERCED FILM AND THERMOPLASTIC FIBRES, ITS USE AND METHOD OF MANUFACTURE

(30) Priorité: 19.04.1993 FR 9304791; 05.05.1993 FR 9305605
(43) Date de publication de la demande: 05.04.1995
(73) Titulaire: GUIAL, 69400 Villefranche sur Saone (FR)
(72) Inventeur: BEZIER, Bernard, F-59130 Lambersart (FR)
(74) Mandataire: Hénnion, Jean-Claude
(86) Numéro de dépôt international: FR9400403
(87) Numéro de publication internationale: WO9424354

(56) Documents cités:
- EP-A- 0 403 187
- WO-A-92/07121
- DE-A- 2 822 588
- US-A- 5 190 812
- DATABASE WPI Section Ch, Week 7842, Derwent Publications Ltd., London, GB; Class A35, AN 78-75495A & JP,A,53 105 573 (TOA NENRYO KOGYO KK) 13 Septembre 1978

## Description

La présente invention concerne un matériau plan composite fibreux dans lequel un film est recouvert de fibres qui sont liées entre elles et également au film. Elle concerne également l'utilisation de ce matériau comme film perméable à l'air et pouvant, de préférence mais non exclusivement, être imperméable aux liquides, notamment dans les articles d'hygiène, par exemple les couches-culottes et les protections féminines. Elle concerne un procédé de fabrication spécialement conçu pour la production dudit matériau plan composite fibreux.

Dans le domaine des couches-culottes, le coussin absorbant est généralement placé sur un film imperméable aux liquides et qui sert à la fois de support audit coussin, permettant son positionnement et sa fermeture sur l'utilisateur, et également de barrière au passage de l'urine. Lorsque l'article est laissé en place sur l'utilisateur alors que le coussin absorbant est imbibé d'urine, il se produit fréquemment des rougeurs voir même des allergies dues à la macération de l'urine au contact de la peau de l'utilisateur. Pour remédier à ce problème, on a déjà proposé d'utiliser comme support du coussin absorbant un film qui soit à la fois imperméable aux liquides et perméable à l'air et à la vapeur d'eau. Ainsi ce film continuerait à faire office de barrière pour l'urine mais permettrait d'assurer un échange gazeux entre l'intérieur de l'article et l'extérieur et donc l'évacuation progressive, sous forme de vapeur d'eau, de l'urine contenue dans le matelas absorbant.

Un tel film, qui présente des propriétés d'imperméabilité aux liquides et de perméabilité à l'air et à la vapeur d'eau, est réalisé le plus souvent à partir d'un matériau microporeux , qui est commercialement connu sous l'appellation GORETEX. Le coût d'un tel matériau est très élevé. De plus selon le demandeur, son toucher n'est pas très agréable.

Dans le domaine des protections féminines, le coussin absorbant est entouré d'un film qui doit permettre le passage des pertes sanguines, tout en évitant leur reflux. On a déjà proposé, pour ce faire, un film perforé dont les perforations se présentent sous forme de cratères, les proéminences, correspondant aux cratères , étant sur la face qui est en contact avec le coussin absorbant. Là encore, le toucher d'un tel matériau n'est pas agréable.

Le but que s'est fixé le demandeur est de proposer un matériau plan composite qui puisse pallier les inconvénients des films précités.

Ce but est parfaitement atteint par le matériau plan composite de l'invention. De manière caractéristique ce matériau est constitué d'un film thermoplastique perforé, qui présente sur une face une multiplicité de cratères de perforation de petites dimensions et dont l'autre face, lisse, est recouverte de fibres majoritairement thermoplastiques ; les fibres sont liées entre elles et au film, de manière sensiblement continue.

Le liage des fibres sur le film ne doit pas donner lieu à des écrasements ponctuels, tels que ceux qui sont décrits dans le document EP 0 403 187 qui provoquent une déformation préjudiciable des cratères et une hétérogénéité dans la nappe fibreuse. Il est à noter que dans ce document la nappe de fibres est liée non pas à la face lisse, comme c'est le cas de la présente invention, mais à la face présentant les cratères.

De préférence les fibres sont liées entre elles et au film y compris au niveau des parois desdits cratères.

La perméabilité à l'air et à la vapeur d'eau est obtenue grâce à la présence des perforations, permettant l'échange gazeux d'une face à l'autre dudit matériau. Le choix des dimensions des perforations, de leur nombre et de leur forme permet, le cas échéant, de maintenir l'imperméabilité aux liquides du film thermoplastique ou d'obtenir une perméabilité aux liquides dans un seul sens de passage. La présence des fibres en surface du film thermoplastique donne au matériau de l'invention un toucher fibreux, plus agréable.

Les fibres recouvrent le film thermoplastique sur la face lisse de celui-ci, c'est-à-dire celle qui correspond aux parois internes des cratères. La notion de paroi interne et externe des cratères apparaîtra plus clairement dans la description qui sera faite ci-après d'un exemple de réalisation.

De préférence, le matériau plan selon l'invention comporte une densité de perforations comprise entre 10 et 100 au cm², la taille desdites perforations étant comprise entre 0,1 et 1,5 millimètre.

Le liage des fibres entre elles et sur le film est obtenu grâce à la thermoplasticité des matières qui les composent. Il est donc nécessaire que les fibres soient en majorité des fibres thermoplastiques, de manière à ce que le matériau composite de l'invention ait une cohésion suffisante entre le film et les fibres.

Le film thermoplastique perforé peut également être recouvert de fibres thermoplastiques liées entre elles et au film, de manière sensiblement continue, sur l'autre face, c'est-à-dire celle qui présente en surface les cratères de perforation. Dans ce cas on a un toucher fibreux, agréable sur les deux faces.

De préférence la matière constitutive du film et des fibres thermoplastiques est la même, par exemple du polyéthylène. Cependant le film peut être dans une autre matière telle que du polyamide ou du polypropylène, ou un mélange de polyéthylène et d'EVA. Les fibres thermoplastiques peuvent être en polypropylène.

Selon une variante, le liage des fibres entre elles et sur le film est obtenu grâce à la présence de colle, en combinaison, avec la thermoplasticité des matières qui composent les fibres et le film. Dans ce cas il est possible de mettre en oeuvre des matières, ayant des points de fusion sensiblement différents.

La quantité de colle doit être relativement faible pour ne pas altérer la souplesse du matériau composite. Elle sera de préférence de 0,2 g/m² à 5 g/m² pour une quantité de fibres de 2 g/m² à 20 g/m².

Ce matériau composite fibreux est utilisé avantageusement comme film respirable dans la fabrication d'articles absorbants d'hygiène notamment de couches-culottes et de protections féminines. Il s'agit en particulier du film respirable servant de support ou d'enveloppe au coussin absorbant.

C'est un autre objet de l'invention que de proposer un premier procédé de fabrication du matériau plan composite fibreux précité. Ce procédé consiste :
a) à disperser une nappe de fibres majoritairement thermoplastiques, sur un film thermoplastique, éventuellement pré-enduit de colle,
b) à présenter l'ensemble ainsi constitué sur une surface pourvue d'orifices,
c) à chauffer cet ensemble à une température proche de la température de ramollissement des matières thermoplastiques constitutives des fibres et du film tout en créant une aspiration à travers les orifices, en sorte d'obtenir le claquage du film au droit desdits orifices.

Le chauffage de l'ensemble film/fibres permet d'obtenir à la fois le ramollissement du film dans la zone correspondant aux orifices mais également d'obtenir le liage des fibres entre elles et du film de manière sensiblement continue, aux points de contact entre ces différents constituants. En particulier on observe que les parois des cratères formés lors du claquage du film sous l'effet de l'aspiration à travers les orifices, peuvent constituer des zones de liaison privilégiées entre les fibres et avec le film.

La dispersion des fibres sous forme de nappe à la surface du film thermoplastique peut être obtenue par tous les moyens, notamment par pulvérisation de fibres selon la technique des fibres coupées ou SPUN ou encore la technique "soufflé-fondu" dénommée MELT-BLOWN.

Selon une version préférée, le procédé de fabrication est un procédé continu et la surface sur laquelle est présenté l'ensemble film/fibres est constituée par la surface périphérique d'un tambour rotatif équipé d'un système interne de mise en dépression.

Le chauffage de l'ensemble film/fibres peut notamment être réalisé à l'aide de panneaux radiants disposés au-dessus de la surface du tambour dans la portion de celui-ci , qui est équipée du système de mise en dépression.

Un matériau plan composite fibreux de l'invention peut encore être obtenu à l'aide d'un deuxième procédé , selon lequel on disperse une nappe de fibres thermoplastiques, sur un film thermoplastique perforé qui présente sur une face une multiplicité de cratères de perforation de petites dimensions, la dispersion des fibres étant réalisée au moins sur l'autre face, lisse, du film alors que les fibres sont à une température suffisante pour qu'elles adhèrent naturellement à la surface du film après refroidissement de celui-ci.

Dans ce cas on obtient un recouvrement uniforme du film par la nappe de fibres et un liage ponctuel des fibres entre elles et au film, grâce à la seule thermoplasticité des matériaux, sur la surface de celui-ci, exclusion faite des cratères.

Pour être certain de l'accrochage des fibres entre elles et sur la surface du film, une variante du procédé précité consiste à pré-enduire la face lisse du film thermoplastique perforé à l'aide d'une colle, résistant à ladite température. De préférence, il s'agit d'une colle qui est également une polymère thermoplastique, applicable par la technique dénommé MELT-BLOWN.

C'est un autre objet de l'invention que de proposer un troisième procédé de fabrication du matériau plan composite fibreux précité. Ce procédé consiste :
a) à extruder un film thermoplastique sur un non-tissé, constitué d'une nappe de fibres majoritairement thermoplastiques,
b) à présenter l'ensemble ainsi constitué sur une surface pourvue d'orifices,
c) à chauffer cet ensemble à une température proche de la température de ramollissement des matières thermoplastiques constitutives des fibres et du film tout en créant une aspiration à travers les orifices, en sorte d'obtenir le claquage du film au droit desdits orifices.

Par rapport au premier procédé précité, dans la première opération on se sert d'un non-tissé et non du film thermoplastique comme support déjà constitué. Il s'avère que dans ce dernier cas, il n'est plus nécessaire d'envisager la pré-enduction de colle, dans la mesure où, grâce à l'application du film en cours d'extrusion sur le non-tissé, on obtient un collage des fibres sur le film très performant.

La présente invention sera mieux comprise à la lecture de la description qui va être faite de deux exemples de réalisation d'un composite film/fibres de polyéthylène perforé, illustrée par le dessin annexé dans lequel :
La figure 1 est une vue schématique en coupe d'un type de composite,
Les figures 2A, 2B, 2C et 2D sont des vues schématiques en coupe de différentes configurations fibreuses dans les zones de cratère, et
Les figures 3 et 4 sont des représentations schématiques de deux installations de fabrication dudit composite, et
La figure 5 est une vue schématique en coupe d'un autre type de composite.

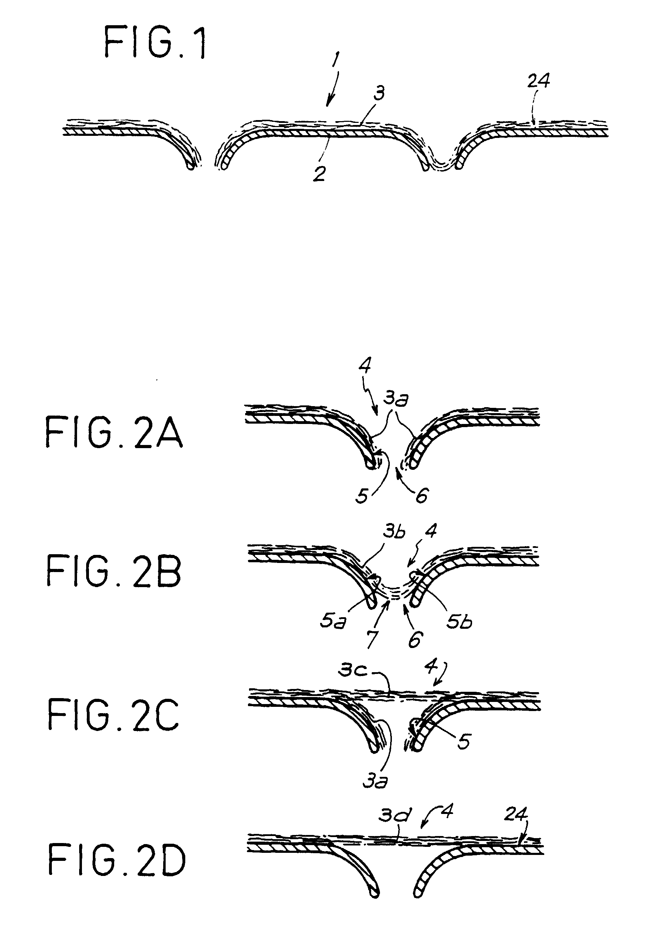

Le matériau composite 1 caractéristique de l'invention est formé d'un film thermoplastique 2 recouvert sur l'une de ses faces de fibres 3, majoritairement thermoplastiques. Le matériau composite 1 comporte une multiplicité de perforations qui ont sensiblement la forme de cratères 4 comme cela apparaît clairement sur la figure 1.

Les fibres 3 sont liées entre elles et au film 2 au moins au niveau des zones lisses 24 du film 2, qui correspondent, sur la face du film qui est opposée aux cratères 4, aux zones planes excluant les parois internes 5 desdits cratères 4. Les fibres 3 sont liées entre elles et au film 2 de manière sensiblement continue, c'est-à-dire sans écrasement ponctuel des fibres ni déformation des cratères.

Les fibres 3 peuvent aussi être liées entre elles et au film 2 au niveau des parois 5 des cratères 4.

Plus précisément, on a représenté schématiquement aux figures 2A, 2B, 2C, 2D quatre configurations que peuvent prendre les fibres 3 se trouvant à l'aplomb du cratère 4.

Selon la première configuration (figure 2A), les fibres 3a sont localisées le long des parois 5 du cratère 4, laissant apparent l'orifice 6 du cratère.

Selon la deuxième configuration (figure 2B) les fibres 3b recouvrent les parois 5 du cratère 4 mais au moins certaines d'entre elles se prolongent d'une paroi Sa à l'autre 5b, obturant au moins partiellement l'orifice 6, dans la zone 7 du cratère 4 qui correspond au sommet dudit cratère.

Selon la troisième configuration (figure 2C), on retrouve comme pour la première configuration des fibres 3a recouvrant les parois 5 du cratère 4. Mais on trouve également d'autres fibres 3c qui s'étendent au-dessus du cratère 4 sensiblement dans le plan où se trouvent les autres fibres dans les zones non perforées du matériau composite 1.

Selon la quatrième configuration (figure 2D), les fibres 3d s'étendent exclusivement au-dessus du cratère 4, sensiblement dans le plan où se trouvent les autres fibres dans les zones lisses 24.

Dans le même matériau composite 1 selon l'invention, on peut bien sûr trouver des cratères dont la couverture fibreuse correspond à l'une ou plusieurs des quatre configurations précitées, voire même d'autres types de configuration, en fonction notamment du mode de fabrication. En particulier il est possible qu'à l'aplomb du cratère 6 il n'y ait pas de fibres, comme dans l'exemple illustré à la figure 2A mais qu'il n'y ait pas non plus de fibres le long des parois internes du cratère 6.

La prédominance de l'une ou l'autre de ces configurations est fonction des conditions de fabrication du matériau composite comme cela ressortira plus clairement de la description qui va être faite ci-après.

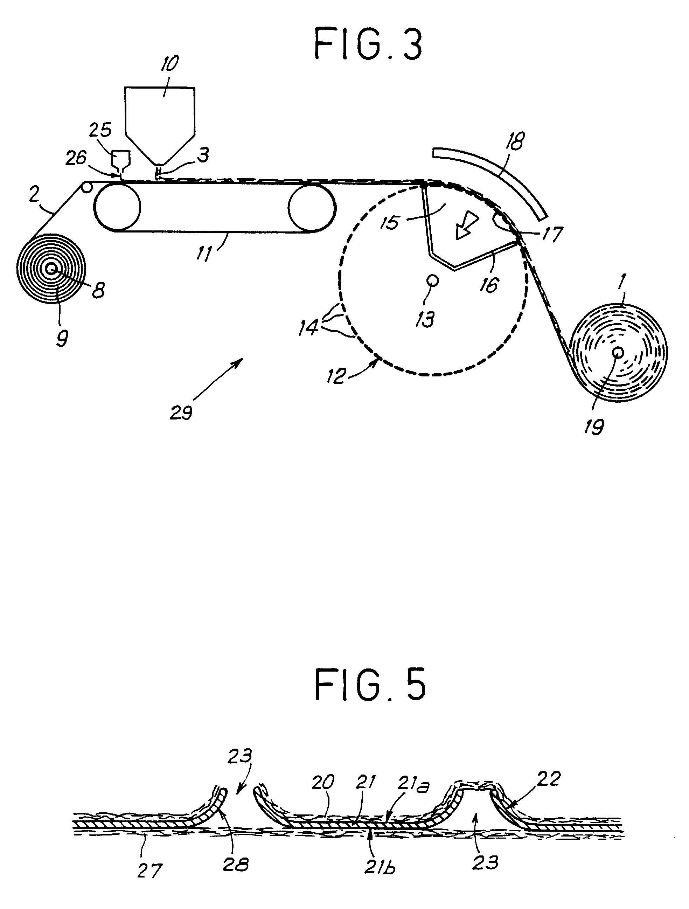

Sur la figure 3 on a représenté de manière très schématique une première installation 29 de fabrication du matériau composite selon les figures 1 et 2.

Cette installation 29 comporte des moyens d'alimentation d'un film thermoplastique 2, par exemple un film de polyéthylène. Ces moyens peuvent consister en un arbre 8 entraîné par des moyens moteurs non représentés, sur lequel est emmanché un rouleau 9 constitué de l'enroulement du film 2.

L'installation 29 comporte sur le parcours de ce film 2 un dispositif 10 de pulvérisation de fibres 3, disposé au-dessus d'un tapis 11 apte à supporter le film 2 pendant cette opération de nappage de fibres 3 sur la face supérieure dudit film 2.

L'installation 29 comporte également un tambour rotatif 12 autour de son axe 13, entraîné en rotation par des moyens connus et non représentés. Ce tambour comporte sur sa périphérie une multiplicité d'orifices 14.

Ce tambour 12 est un cylindre creux à l'intérieur duquel est agencée une chambre d'aspiration 15. Cette chambre 15 sensiblement étanche est délimitée par un montant intérieur 16 qui est fixe et par une portion 17 du tambour 12. Dans cette chambre 15 débouchent des moyens connus et non représentés d'aspiration, par exemple un ventilateur.

L'installation 29 comporte également un ensemble de panneaux radiants 18, disposés en arc de cercle au-dessus de la portion 17 du tambour 12 en regard de la chambre d'aspiration 15.

L'installation 29 comporte enfin des moyens de réception 19 du matériau composite 1.

Le fonctionnement de l'installation 29 est le suivant. Le film thermoplastique 2 est disposé sur le tapis 11. Le dispositif de pulvérisation 10 réalise sur la face supérieure du film 2 une nappe continue et régulière de fibres 3 qui sont simplement déposées sur le film 2, sans aucune liaison particulière avec ledit film. L'ensemble constitué du film 2 et de la nappe de fibres 3 est amené sur le tambour 12 et est appliqué sur celui-ci sensiblement sur toute la portion 17 en regard de la chambre d'aspiration 15 avant d'être enroulé sur le dispositif 19 de réception. Pendant que l'ensemble film/fibres se déplace sur le tambour 12, pendant la rotation de l'axe 13 de celui-ci, il est soumis simultanément à l'action de chauffage des panneaux radiants 18 et à l'action d'aspiration de la chambre 15.

L'action de chauffage a pour effet de porter l'ensemble film/fibres à une température proche de la température de ramollissement des matières thermoplastiques constitutives d'une part du film 2 et d'autre part des fibres 3.

L'action d'aspiration, du fait de la présence sur le tambour 12 des orifices 14, a pour effet non seulement de plaquer le film 2 sur la surface dudit tambour 12 mais de plus de créer des perforations dans l'ensemble film/fibres. En effet le film thermoplastique 2 étant dans un état proche du ramollissement, les forces d'aspiration provoquent une déformation des zones du film qui se trouvent au-dessus des orifices 14 ; cette déformation, similaire à une boursouflure, se poursuit par un claquage de celle-ci qui prend alors la forme d'un cratère 4. Il y a donc un percement du film 2 sous la simple action mécanique due à l'aspiration.

S'agissant des fibres 3 qui se trouvent situées à la surface du film 2, elles sont également ramollies lorsque se réalise la formation des cratères 4 et sont plus ou moins entraînées lors de la déformation du film 2. Cet entraînement plus ou moins important des fibres 3 est fonction de certaines conditions opératoires, en particulier de la température de chauffage, de la pression qui aura pu éventuellement être exercée pour appliquer les fibres 3 sur le film 2, de la longueur des fibres 3, de la proportion de fibres non thermoplastiques dans le mélange fibreux, de l'écart entre la température de ramollissement des fibres 3 et celle du film 2 ... Ainsi lors de la création des boursouflures, une quantité plus ou moins importante de fibres va rester liée aux parois internes de la boursouflure, ce qui entraîne qu'après le claquage et la formation du cratère 4 lesdites fibres recouvrent les parois internes 5 dudit cratère 4. C'est ce qui explique les différentes configurations qui sont illustrées aux figures 2A, 2B, 2C et 2D.

Il peut être préférable de disposer sur le parcours du film thermoplastique 2, en amont du dispositif 10 de pulvérisation de fibres 3, un dispositif 25 de projection de colle 26, apte à déposer une quantité régulière et faible de colle sur toute la face supérieure du film 2. Il peut s'agir notamment d'un dispositif du type MELT-BLOWN projetant une colle 26 sous forme d'une résine thermoplastique adhésive.

L'apport de colle doit être en quantité suffisante pour parfaire l'accrochage des fibres entre elles et sur le film tout en étant suffisamment peu important pour éviter une altération de la souplesse du matériau de l'invention. Il est aussi fonction de la quantité de fibres 3 disposées sur le film 2 ; la fourchette préférée de 0,2 à 5 g/m² de colle correspond à la fourchette de 2 à 20 g/m² de fibres.

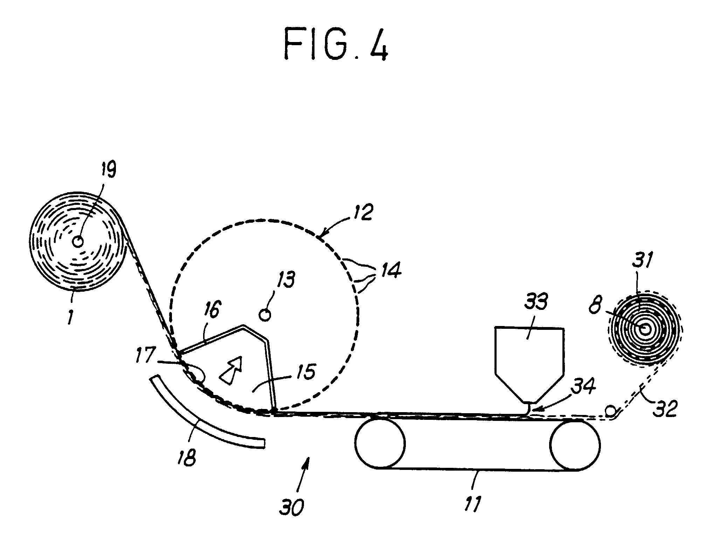

Selon une variante de réalisation, le matériau composite selon l'invention peut être fabriqué sur une seconde installation 30 représentée schématiquement à la figure 4. Par simplification on a gardé les mêmes références pour les éléments communs aux deux installations 29 et 30.

Sur l'arbre 8 est emmanché un rouleau 31 constitué de l'enroulement d'un non-tissé 32, qui est constitué d'une nappe de fibres majoritairement thermoplastiques.

L'installation 30 comporte sur le parcours de ce non-tissé 32 un dispositif 33 d'extrusion d'un film thermoplastique 34 ; ce dispositif d'extrusion 33 est disposé au-dessus d'un tapis 11 apte à supporter le non-tissé 32 pendant que se réalise l'extrusion du film 34 sur la face supérieure dudit non-tissé 32.

L'installation 30 comporte le même tambour rotatif 12, perforé est avec aspiration et les mêmes panneaux radiants 18, que ce qui a été décrit pour l'installation 29. Cependant le tambour 12 devant être disposé de telle sorte que ce soit le film thermoplastique 34 qui vienne en contact avec la portion 17 du tambour 17 en regard de la chambre 15, sa disposition se trouve inversée, comparativement à l'installation 29.

Le fonctionnement de l'installation 30 est le suivant. Le non-tissé 32 est disposé sur le tapis 11. Le dispositif 33 réalise l'extrusion du film thermoplastique 34 qui est immédiatement appliqué sur la face supérieure du non-tissé 32, alors qu'il n'est pas encore totalement refroidi. On obtient déjà un certain liage des fibres du non-tissé 32 et du film 34.

Les autres étapes sont identiques à celles qui ont été décrites précédemment pour l'installation 29.

Dans ce cas, du fait que le film thermoplastique est extrudé sur une nappe de fibres déjà consolidée, on remarque, après passage sur le tambour rotatif, que les fibres du non-tissé dans les zones surplombant les cratères non seulement ne sont généralement pas liées au film au niveau des parois internes des cratères mais aussi sont écartées les unes des autres et forment des trous au droit des cratères.

Bien sûr il serait possible d'obtenir le matériau composite de l'invention selon d'autres techniques que les procédés qui viennent d'être décrits, par exemple en mettant en oeuvre la technique d'aiguilletage. Cependant le procédé précité présente l'avantage de pouvoir, en réglant les conditions opératoires,obtenir une variété de produits différents basés sur le même concept, variété beaucoup plus importante qu'en mettant en oeuvre la technique d'aiguilletage.

Il est également possible de réaliser la dispersion des fibres sur le film préalablement perforé. Dans ce cas la dispersion des fibres doit être faite sur la face du film qui ne comporte pas de cratères et les fibres étant dans les conditions de température telles que, après refroidissement, elles adhèrent entre elles et sur les zones lisses 24 du film. Ces conditions sont obtenues en réalisant l'extrusion directe des fibres au-dessus du film en défilement continu et la projection desdites fibres sur le film, alors qu'elles sont encore à une température supérieure à la température de fusion du matériau thermoplastique dont elles sont constituées. Le matériau obtenu aura principalement la configuration de la figure 2D.

Dans ce cas, il est aussi préférable de pré-enduire à l'aide de colle la face du film sur laquelle sont projetées des fibres. La colle sera par exemple une résine adhésive thermoplastique, réagissant à la température à laquelle les fibres viennent en contact avec le film.

Dans l'exemple précité, seule la face du film correspondant aux parois internes 5 des cratères 4 comportait une couverture fibreuse. Ceci n'est pas limitatif de l'invention. On a représenté à la figure 4 un exemple de réalisation dans lequel une première surface fibreuse 20 se trouve sur la face 21a du film 21 qui correspond aux parois externes 22 des cratères 23 et une seconde surface fibreuse 27 se trouve sur la face 21b du film 21 qui correspond aux parois internes 28 des cratères 23. Un tel matériau peut être réalisé par un premier passage sur l'installation à la figure 3 puis par pulvérisation des fibres sur l'autre face conformément au procédé d'extrusion directe sur le film.

Le matériau composite de l'invention est avantageusement mis en oeuvre dans la confection d'articles d'hygiène absorbants jetables. Dans le cas de couches-culottes, il est utilisé comme feuille support du coussin absorbant, les cratères étant de préférence tournés vers l'intérieur de l'article. Ainsi ledit support reste imperméable aux liquides et sert donc de barrière au passage de l'urine mais, grâce aux perforations, il est perméable au gaz et permet donc un échange de l'air et de la vapeur d'eau entre l'intérieur et l'extérieur de l'article.

Dans le cas de protections féminines, il est utilisé comme feuille entourant le coussin absorbant, les cratères étant tournés vers le coussin absorbant. Les formes et dimensions des perforations sont telles que le matériau composite de l'invention est perméable aux liquides dans le sens allant vers le coussin absorbant mais ne l'est pas dans l'autre sens.

Dans les deux cas, la présence des fibres confère au matériau de l'invention un toucher agréable, très éloigné du toucher plastique des films servant actuellement de support ou d'enveloppe au coussin absorbant.

La taille et la densité des perforations sont fonction de l'application envisagée. Dans un exemple précis dans le domaine de l'hygiène, pour un film d'environ 80 micromètres d'épaisseur, on avait une densité de l'ordre de 50 cratères au cm², avec un pourcentage d'ouverture de l'ordre de 25% (rapport entre les surfaces des sommets des cratères et la surface totale du film). De préférence le film fait de 3 à 40 g/m².

Il peut être avantageux que le film thermoplastique ait une certaine élasticité naturelle. Dans ce cas, de préférence, le liage des fibres sur le film a lieu alors que le film se présente à l'état étiré. Une fois le matériau plan perforé fibreux réalisé, le film retrouve son état initial non étiré ; la couverture fibreuse a alors plus de volume, de gonflant, et ne risque pas de se rompre lors de la mise en extension éventuelle du matériau.

Les fibres constituant le non-tissé peuvent être des polymères comme par exemple le polypropylène ou le polyester, ou un mélange de différents polymères, ou un mélange de fibres synthétiques avec des fibres naturelles ou artificielles comme par exemple la viscose. Les fibres bi-composantes peuvent être aussi une solution pour construire un matelas fibreux.

La présente invention n'est pas limitée aux modes de réalisation qui viennent d'être décrits à titre d'exemples non exhaustifs. En particulier le film thermoplastique peut être en polyéthylène, en polyamide, en EVA, en polypropylène ou un mélange de ces matières en coextrusion ou en monocouche.

## Revendications

1. Matériau plan composite constitué d'un film (2) thermoplastique recouvert de fibres majoritairement thermoplastiques liées entre elles et au film, ledit film présentant sur une face une multiplicité de cratères (4) de petites dimensions, caractérisé en ce que le film comporte une face lisse et en ce que les fibres sont liées au film de manière sensiblement continue sur la dite face lisse.

2. Matériau selon la revendication 1 caractérisé en ce que les fibres (3) sont liées entre elles et au film, y compris au niveau des parois internes (5) des cratères (4).

3. Matériau selon la revendication 1 ou 2, caractérisé en ce qu'il comporte une densité de perforations comprise entre 10 et 100 au cm², la taille desdites perforations étant comprise entre 0,1 et 1,5 millimètre.

4. Matériau selon la revendication 1 caractérisé en ce que le film thermoplastique (21) est recouvert de fibres (20) majoritairement thermoplastiques liées entre elles et au film également sur l'autre face (21a) qui présente des cratères (23) de perforations.

5. Utilisation du matériau plan composite selon l'une des revendications 1 à 4 comme film respirable dans la fabrication d'articles absorbants d'hygiène notamment de couches-culottes et protections féminines.

6. Procédé de fabrication du matériau plan composite fibreux selon la revendication 1 caractérisé en ce qu'il consiste :
a) à disperser une nappe de fibres (3) majoritairement thermoplastiques, sur un film thermoplastique (2),
b) à présenter l'ensemble ainsi constitué sur une surface (12) pourvue d'orifices (14),
c) à chauffer cet ensemble à une température proche de la température de ramollissement des matières thermoplastiques constitutives des fibres (3) et du film (2) tout en créant une aspiration à travers les orifices, en sorte d'obtenir le claquage du film au droit desdits orifices.

7. Procédé de fabrication du matériau plan composite fibreux selon la revendication 1 caractérisé en ce qu'il consiste à disperser une nappe de fibres thermoplastiques, sur un film thermoplastique perforé qui présente sur une face une multiplicité de cratères de perforation de petites dimensions, la dispersion des fibres étant réalisée au moins sur l'autre face, lisse, du film alors que les fibres sont à une température suffisante pour qu'elles adhèrent naturellement à la surface du film après refroidissement de celui-ci.

8. Procédé selon l'une des revendications 6 ou 7 caractérisé en ce qu'il comprend une étape préalable de pré-enduction de colle sur la face du film (2) sur laquelle sont dispersées les fibres (3).

9. Procédé selon l'une des revendications 8 ou 9 caractérisé en ce que la colle est une résine adhésive thermoplastique, réagissant à la température à laquelle les fibres viennent en contact avec le film.

10. Procédé selon la revendication 9 caractérisé en ce que la pré-enduction est réalisé par la technique MELT-BLOWN à raison de 0,2 à 5 g/m² de colle.

11. Procédé selon l'une des revendications 6 ou 7 caractérisé en ce qu'il consiste à étirer le film thermoplastique lors de la dispersion des fibres sur ledit film.

12. Procédé de fabrication du matériau plan composite fibreux selon la revendication 1 caractérisé en ce qu'il consiste :
a) à extruder un film thermoplastique sur un non-tissé constitué d'une nappe de fibres majoritairement thermoplastiques,
b) à présenter l'ensemble ainsi constitué sur une surface (12) pourvue d'orifices (14),
c) à chauffer cet ensemble à une température proche de la température de ramollissement des matières thermoplastiques constitutives des fibres (3) et du film (2) tout en créant une aspiration à travers les orifices, en sorte d'obtenir le claquage du film au droit desdits orifices.

13. Procédé selon l'une des revendications 6 ou 12 caractérisé en ce que la surface sur laquelle est présenté l'ensemble film/fibres est constituée par la surface périphérique d'un tambour rotatif (12) équipé d'un système interne de mise en dépression.

## Patentansprüche

1. Verbund-Flächenmaterial, das aus einer Folie (2) gebildet ist, die mit überwiegend thermoplastischen Fasern abgedeckt ist, die miteinander und mit der Folie verbunden sind, wobei die genannte Folie auf der einen Fläche eine Vielzahl von Kratern bzw. Einstülpungen (4) mit kleinen Abmessungen aufweist, dadurch gekennzeichnet, daß die Folie eine glatte Fläche aufweist, und daß die Fasern mit der Folie auf der genannten glatten Fläche im wesentlichen durchgehend verbunden sind.

2. Material nach Anspruch 1, dadurch gekennzeichnet, daß die Fasern (3) untereinander und mit der Folie verbunden sind, wobei hier auch der Bereich der Innenwände (5) der Einstülpungen (4) umfaßt ist.

3. Material nach Anspruch 1 oder 2, dadurch gekennzeichnet, daß es eine Perforierungsdichte aufweist, die zwischen 10 und 100 pro cm² liegt, wobei die Größe der genannten Perforierungen zwischen 0,1 und 1,5 mm liegt.

4. Material nach Anspruch 1, dadurch gekennzeichnet, daß die thermoplastische Folie (21) mit überwiegend thermoplastischen Fasern (20), die miteinander und der Folie verbunden sind, auch auf der anderen Fläche (21a) abgedeckt ist, die Perforierungs-Einstülpungen (23) darbietet.

5. Verwendung des Verbund-Flächenmaterials nach einem der Ansprüche 1 bis 4 als atmungsfähige Folie bei der Herstellung bei der Herstellung von absorbierenden Hygieneartikeln, insbesondere Höschenwindeln und Schutzeinlagen für Frauen.

6. Verfahren zur Herstellung des faserigen Verbund-Flächenmaterials nach Anspruch 1, dadurch gekennzeichnet, daß es aus dem folgenden besteht:
a) Verteilen einer Lage aus überwiegend thermoplastischen Fasern (3) auf einer thermoplastischen Folie (2),
b) Darbieten der so gebildeten Anordnung einer Oberfläche (12), die mit Öffnungen (14) versehen ist, und
c) Erwärmen dieser Anordnung auf eine Temperatur nahe der Erweichungstemperatur der thermoplastischen Materialien, die die Fasern (3) und die Folie (2) bilden, während man gleichzeitig eine Saugwirkung durch die Öffnungen hindurch derart erzeugt, daß man das Anhaften der Folie längs der genannten Öffnungen erzielt.

7. Verfahren zur Herstellung des faserigen Verbund-Flächenmaterials nach Anspruch 1, dadurch gekennzeichnet, daß es daraus besteht, eine Lage aus thermoplastischen Fasern auf einer perforierten, thermoplastischen Folie zu verteilen, die auf der einen Fläche eine Vielzahl von eingestülpten Perforierungen mit kleinen Abmessungen darbietet, wobei die Verteilung der Fasern mindestens auf der anderen, glatten Fläche der Folie bewirkt wird, während die Fasern eine ausreichende Temperatur aufweisen, so daß sie auf natürliche Weise an der Oberfläche der Folie nach deren Abkühlung anhaften.

8. Verfahren nach einem der Ansprüche 6 oder 7, dadurch gekennzeichnet, daß es einen vorausgehenden Schritt des vorherigen Beschichtens der Fläche der Folie (2) mit Kleber aufweist, auf der die Fasern (3) verteilt werden.

9. Verfahren nach einem der Ansprüche 8 oder 9, dadurch gekennzeichnet, daß der Kleber ein klebendes, thermoplastisches Harz ist, das bei der Temperatur reagiert, bei der die Fasern in Berührung mit der Folie gelangen.

10. Verfahren nach Anspruch 9, dadurch gekennzeichnet, daß die vorausgehende Beschichtung durc die Melt-Blow-Technik bzw. Schmelzblasetechnik mit einer Klebermenge von 0,2 bis 5g/m² durchgeführt wird.

11. Verfahren nach einem der Ansprüche 6 oder 7, dadurch gekennzeichnet, daß es darin besteht, die thermoplastische Folie während der Verteilung der Fasern auf der genannten Folie zu strecken.

12. Verfahren zur Herstellung des faserigen Verbund-Flächenmaterials nach Anspruch 1, dadurch gekennzeichnet, daß es aus dem folgenden besteht:
a) Aufextrudieren einer thermoplastischen Folie auf einen nicht-gewebten Stoff, der von einer Schicht aus überwiegend thermoplastischen Fasern gebildet ist,
b) Aufbringen der so gebildeten Anordnung auf eine (12), die mit öffnungen (14) versehen ist, und
c) Erwärmen dieser Anordnung auf eine Temperatur nahe der Erweichungstemperatur der thermoplastischen Materialien, die die Fasern (3) und die Folie (2) bilden, während man gleichzeitig eine Saugwirkung durch die Öffnungen hindurch derart erzeugt, daß man ein Anhaften der Folie längs der genannten Öffnungen erzielt.

13. Verfahren nach einem der Ansprüche 6 oder 12, dadurch gekennzeichnet, daß die Oberfläche, auf welche die Anordnung aus Folie und Fasern aufgebracht wird, durch die Umfangsoberfläche einer rotierenden Trommel (12) gebildet ist, die im Inneren mit einem System zum Erzeugen von Unterdruck ausgestattet ist.

## Claims

1. A composite plane material constituted by a thermoplastic film (2) covered in fibers, most of which are thermoplastic and that are bonded to one another and to the film, said film having a multiplicity of craters (4) of small dimensions on one of its faces, the material being characterised in that the film comprises a smooth face and in that the fibers are bonded to the film in substantially continuous manner over said smooth face.

2. A material according to claim 1, characterised in that the fibers (3) are bonded to one another and to the film, even on the inside walls (5) of the craters (4).

3. A material according to claim 1 or 2, characterised in that it includes perforations at a density lying in the range 10 per cm² to 100 per cm², the size of said perforations lying in the range 0.1 mm to 1.5 mm.

4. A material according to claim 1, characterised in that the thermoplastic film (21) is covered with fibers (20) most of which are thermoplastic and that are bonded to one another and to the film also on the other face (21a) that has perforation craters (23).

5. The use of the composite plane material according to one of claims 1 to 4, as a breathing film in the manufacture of absorbent hygiene articles, in particular diapers and sanitary napkins.

6. A method of manufacturing the composite plane fiber material according to claim 1, characterised in that it consists:
a) in dispering a sheet of fibers (3) most of which are thermoplastic on a thermoplastic film (2);
b) in presenting the assembly made up in this way on a surface (12) that is provided with orifices (14);
c) in heating said assembly to a temperature close to the softening temperature of the thermoplastic materials constituting the fibers (3) and the film (2) while simultaneously establishing suction through the orifice, so as to cause the film over said orifices to puncture.

7. A method of manufacturing a composite plane fiber material according to claim 1, characterised in that it consists in dispersing a sheet of thermoplastic fibers on a perforated thermoplastic film having a multiplicity of perforation craters of small dimensions on one of its faces, the fibers being dispersed at least over the smooth, other face of the film while the fibers are at a temperature that is high enough to cause them to adhere naturally to the surface of the film after it has cooled.

8. A method according to one of claims 6 or 7, characterised in that it includes a prior step of pre-coating adhesive on the face of the film (2) onto which the fibers (3) are dispersed.

9. A method according to one of claims 8 or 9, characterised in that the adhesive is a thermoplastic adhesive resin that reacts at the temperature at which the fibers come into contact with the film.

10. A method according to claim 9, characterised in that the pre-coating is implemented by the melt-blown technique at a rate of 0.2 g/m² to 5 g/m² of adhesive.

11. A method according to one of claims 6 or 7, characterised in that it consists in stretching the thermoplastic film while the fibers are being dispersed on said film.

12. A method of manufacturing a composite plane fiber material according to claim 1, characterised in that it consists:
a) in extruding a thermoplastic film on a non-woven fabric constituted by a sheet of fibers most of which are thermoplastic;
b) in presenting the assembly constituted in this way onto a surface (12) that is provided with orifices (14);
c) in heating said assembly to a temperature close to the softening temperature for the thermoplastic materials constituting the fibers (3) and the film (2) while simultaneously establishing suction through the orifices so as to cause the film overlying said orifices to puncture.

13. A method according to one of claims 6 or 12, characterised in that the surface on which the film/fiber assembly is presented is constituted by the peripheral surface of a rotary drum (12) fitted with an internal suction system.
